(19) Europäisches Patentamt
European Patent Office
Office européen des brevets

(11) Veröffentlichungsnummer : **0 304 777 B1**

# (12) EUROPÄISCHE PATENTSCHRIFT

(45) Veröffentlichungstag der Patentschrift : **04.09.91 Patentblatt 91/36**

(51) Int. Cl.$^5$ : **C07D 311/72**

(21) Anmeldenummer : **88113307.8**

(22) Anmeldetag : **17.08.88**

## (54) Verfahren zur Herstellung von Chromanderivaten bzw. von d-alpha-Tocopherol.

(30) Priorität : **25.08.87 CH 3244/87**

(43) Veröffentlichungstag der Anmeldung : **01.03.89 Patentblatt 89/09**

(45) Bekanntmachung des Hinweises auf die Patenterteilung : **04.09.91 Patentblatt 91/36**

(84) Benannte Vertragsstaaten : **AT BE CH DE FR GB IT LI NL**

(56) Entgegenhaltungen : **EP-A- 0 058 945**

(73) Patentinhaber : **F. HOFFMANN-LA ROCHE AG**
**Postfach 3255**
**CH-4002 Basel (CH)**

(72) Erfinder : **Barner, Richard, Dr.**
**Traubenweg 16**
**CH-4108 Witterswil (CH)**
Erfinder : **Hübscher, Josef**
**Säspelstrasse 1**
**CH-4208 Nunningen (CH)**

(74) Vertreter : **Urech, Peter, Dr. et al**
**Grenzacherstrasse 124 Postfach 3255**
**CH-4002 Basel (CH)**

**EP 0 304 777 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von Chromanderivaten, welche als Zwischenprodukte für die Herstellung von d-α-Tocopherol (natürliches Vitamin E) geeignet sind, sowie ein Verfahren zur Herstellung von d-α-Tocopherol selbst. Weiterhin betrifft die Erfindung neue Zwischenprodukte in diesem Verfahren.

Es sind bereits einige Verfahren zur Herstellung von natürlichem Vitamin E bekannt, welche jedoch technisch nur von begrenztem Interesse sind, so dass natürliches Vitamin E bis heute nahezu ausschliesslich aus natürlichen Quellen extrahiert wird.

Es bestand somit ein Bedürfnis nach einem technisch realisierbaren Verfahren, gemäss welchem natürliches Vitamin E in guter Ausbeute und mit hoher optischer Reinheit erhalten werden kann. Dies ist nunmehr mittels des erfindungsgemässen Verfahrens möglich.

Dieses Verfahren ist dadurch gekennzeichnet, dass man Phytenal, also die Verbindung der allgemeinen Formel

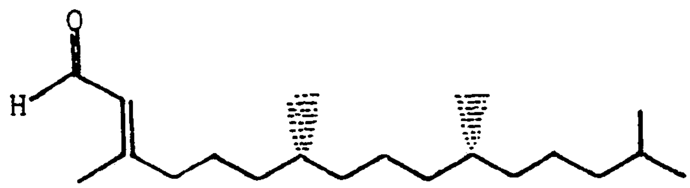

I

mit einer Verbindung der allgemeinen Formel

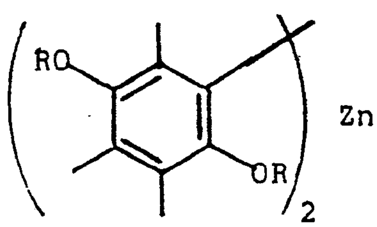

II

worin OR eine hydrolysierbare Aethergruppe darstellt, unter Verwendung eines chiralen Katalysators der Formeln

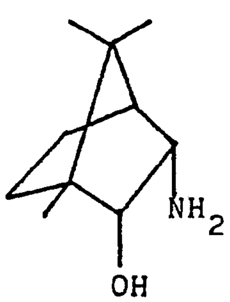

III oder

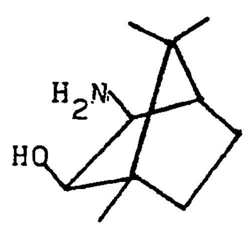

IV

umsetzt, und dass man, gewünschtenfalls, eine so erhaltene Verbindung der allgemeinen Formel

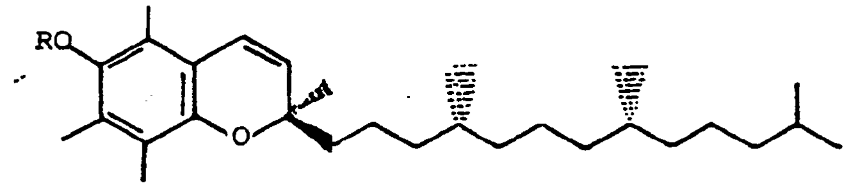

V

worin OR die obige Bedeutung hat, in d-α-Tocopherol überführt.

Der Rest -OR bedeutet im Rahmen der vorliegenden Erfindung eine durch Hydrolyse spaltbare Gruppe ; R ist demnach zweckmässigerweise Silyl oder Alkoxymethyl, beispielsweise Methoxymethyl, oder auch Tetrahydropyranyl. Weiterhin bedeutet das Zeichen "►", dass sich der entsprechende Rest oberhalb der Molekülebene befindet, während das Zeichen "ıııı.." bedeutet, dass sich der entsprechende Rest unterhalb der Molekülebene befindet.

Die Umsetzung von Phytenal mit einer Verbindung der allgemeinen Formel II erfolgt zweckmässig dergestalt, dass man zunächst eine Verbindung der Formel II gemäss

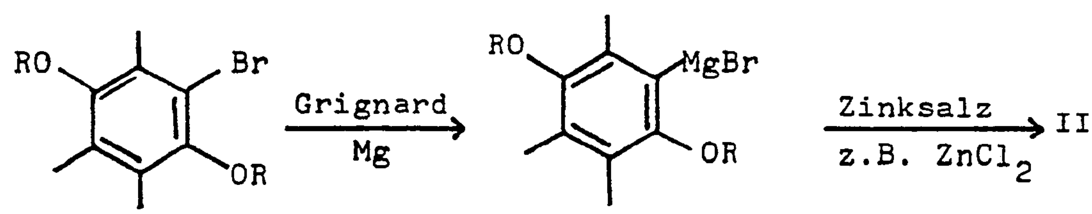


VI VII

in an sich bekannter Weise (siehe z.B. L.I. Smith et al., J. Amer. Chem. Soc. 59, 673 (1937), R. Barner et al., Helv. 62, 2384 (1979), R.A. Grey, J. Org. Chem. 49, 2288 (1984), Houben-Weyl, Methoden der organischen Chemie, 2. Aufl. (1924), Band 4, 754 ff.) in Lösung herstellt.

Es erfolgt hierauf die Zugabe der Verbindung III oder IV.

Ein zweckmässiges Verhältnis von II : III bzw. IV ist dasjenige von wenigen Molprozenten, beispielsweise 2 Mol%.

Anschliessend erfolgt die Zugabe von Phytenal.

Ein zweckmässiges Verhältnis von I : II ist dasjenige von ca. 1 : 1.

Als inerte organische Lösungsmittel können im Rahmen der vorliegenden Erfindung die üblicherweise bei metallorganischen Reaktionen verwendeten Lösungsmittel verwendet werden. Als Beispiele können hier genannt werden Aether, insbesondere cyclische Aether wie Tetrahydrofuran oder Dioxan oder auch Gemische dieser Aether mit aliphatischen oder aromatischen Kohlenwasserstoffen, wie insbesondere Pentan, Hexan, Benzol, Toluol und dergleichen.

Der Katalysator III bzw. IV kann als solcher oder in Polymer-gebundener Form eingesetzt werden.

Die Verbindungen der Formel V sind neu und ebenfalls Gegenstand der vorliegenden Erfindung. Die Ueberführung einer derartigen Verbindung in d-α-Tocopherol durch Hydrierung der Doppelbindung und Abspaltung der Schutzgruppe kann in an sich bekannter Weise erfolgen ; die Hydrierung also z.B. mit Wasserstoff katalytisch oder mittels Alkalimetall/$C_2H_5OH$, die Entfernung der Schutzgruppe durch saure Hydrolyse oder Solvolyse.

Die katalytische Hydrierung erfolgt zweckmässigerweise mineralsauer (gleichzeitige Abspaltung der Schutzgruppe). Der geeignete Katalysator ist insbesondere Pt oder Pd.

Die Reduktion mittels Alkalimetall, insbesondere mittels Natrium in Aethanol, erfolgt zweckmässigerweise bei erhöhter Temperatur, insbesondere bei Rückflusstemperatur ; siehe auch Methods in Enzymology, Vol. XVIII, Vitamins and Coenzymes, Part C, D.B. McCormick ad L.D. Wright, Academic Press N.Y., 1971, Seite 272.

Das nachfolgende Beispiel illustriert die Erfindung und stellt keinerlei Einschränkung hiervon dar.

Beispiel

1.6 g (5 mMol) Bromtrimethyldimethoxybenzol werden in bekannter Weise mit Magnesium in trockenem Tetrahydrofuran ins Grignardreagens übergeführt, durch Zugabe von 240 mg (2,5 mMol) Zinkchlorid wird das Zinkderivat erhalten, was 30 Minuten erfordert. Es wird Toluol zugegeben und bei Raumtemperatur 15 mg (0.09 mMol) endo-Aminoborneol. Anschliessend erfolgt die Zugabe von 1,2 g (4.1 mMol) (7R, 11R)-E-3,7,11,15-Tetramethyl-hexadec-2-en-1-al, und nach 16-stündigem Rühren wird mit wässriger Ammoniumchloridlösung hydrolysiert. Es wird mit Essigester extrahiert, getrocknet und eingeengt. Das erhaltene Dehydrotocopherol (Methoxymethyläther) wird in MeOH/1% HCl über PdC (5%) hydriert (gleichzeitige Entfernung der Schutzgruppe). Es wird eingeengt und anschliessend an Kieselgel mit Toluol chromatographiert. Es resultieren 0,37 g (55% Ausbeute) d-α-Tocopherol. Die optische Reinheit wird durch gaschromatographische Analyse des Methyläthers bestimmt.

## Patentansprüche

1. Verfahren zur Herstellung von Chromanderivaten bzw. von d-α-Tocopherol, dadurch gekennzeichnet, dass man Phytenal mit einer metallorganischen Verbindung der allgemeinen Formel

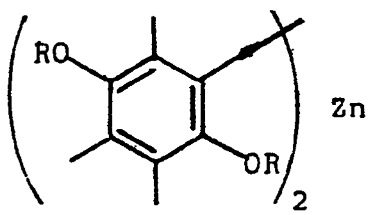

II

worin OR eine hydrolysierbare Aethergruppe darstellt, unter Verwendung eines chiralen Katalysators der Formeln

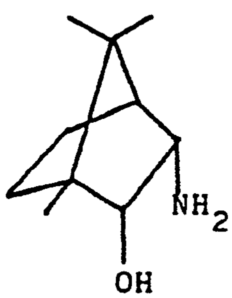

III

oder

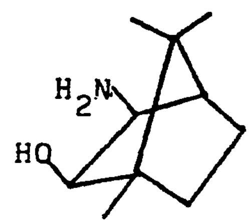

IV

umsetzt, und, gewünschtenfalls, eine so erhaltene Verbindung der allgemeinen Formel

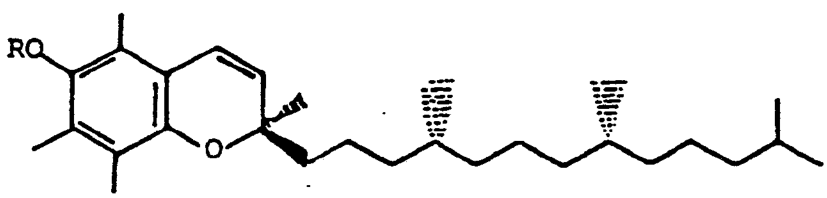

V

unter Abspaltung von R zu d-α-Tocopherol hydriert.

2. Verfahren nach Anspruch 1, dadurch gekennzeichnet, dass R für Methoxymethyl oder Tetrahydropyranyl steht.

3. Verfahren nach Anspruch 1 oder 2, dadurch gekennzeichnet, dass man die Verbindung II in situ herstellt.

4. Verbindungen der Formel

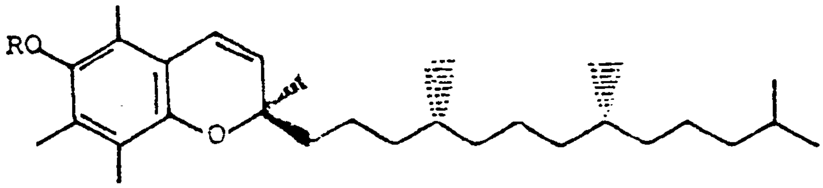

V

worin OR eine hydrolysierbare Aethergruppe darstellt.

5. 3,4-Dehydro-(2R,4'R,8'R)-α-tocopherylmethoxymethyläther.

## Claims

1. A process for the manufacture of chromane derivatives and of d-α-tocopherol, characterized by reacting phytenal with a metal-organic compound of the general formula

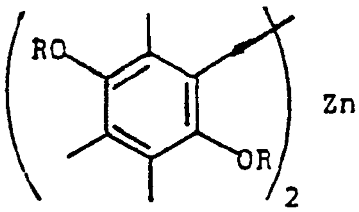

II

wherein OR represents a hydrolyzable ether group, using a chiral catalyst of the formula

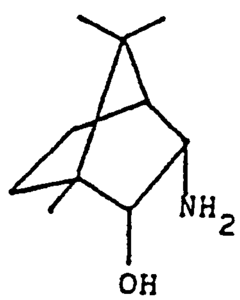

III or

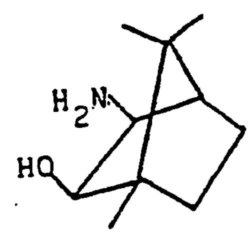

IV

and, if desired, hydrogenating a thus-obtained compound of the general formula

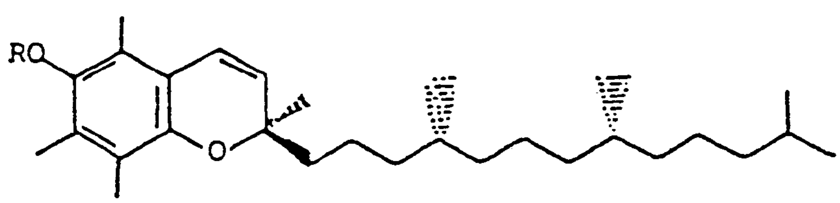

V

with the cleavage of R to give d-α-tocopherol.

2. A process according to claim 1, characterized in that R stands for methoxymethyl or tetrahydropyranyl.

3. A process according to claim 1 or 2, characterized in that the compound II is prepared in situ.

4. Compounds of the formula

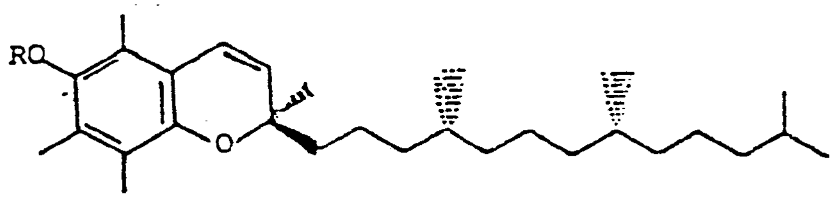

V

wherein OR represents a hydrolyzable ether group.

5. 3,4-Dehydro-(2R,4′R,8′R)-α-tocopheryl methoxy-methyl ether.

**Revendications**

1. Procédé de préparation de dérivés du chromanne ou du d-alpha-tocophérol, caractérisé en ce que l'on fait réagir le phyténal avec un composé organométallique de formule générale

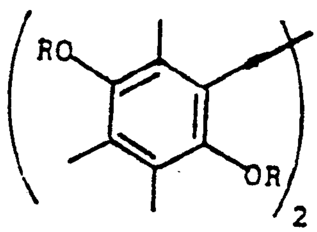

II

dans laquelle OR représente un groupe éther hydrolysable, avec utilisation d'un catalyseur chiral de formule

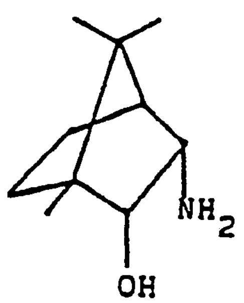

III

ou

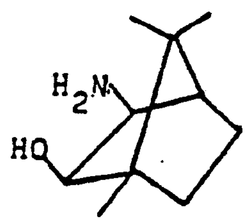

IV

et, si on le désire, on convertit le composé ainsi obtenu, répondant à la formule générale

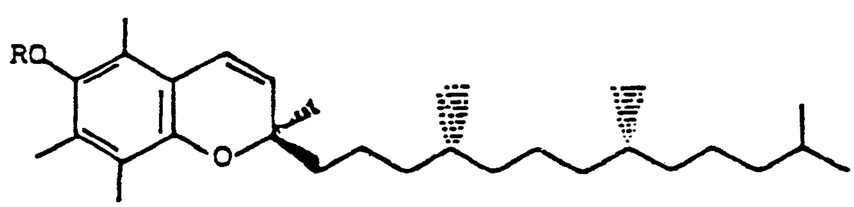

V

avec scission de R, en le d-alpha-tocophérol.

2. Procédé selon la revendication 1, caractérisé en ce que R représente un groupe méthoxy-méthyle ou tétrahydropyrannyle.

3. Procédé selon la revendication 1 ou 2, caractérisé en ce que l'on prépare le composé II in situ.

4. Composés de formule

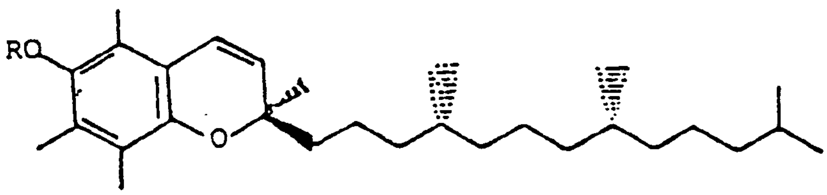 V

dans laquelle OR représente un groupe éther hydrolysable.

5. L'éther 3,4-déshydro-(2R,4'R,8'R)-alpha-tocophérylméthoxyméthylique.